# EUROPEAN PATENT APPLICATION

(11) **EP 1 806 089 A1**
(43) Date of publication of application: **11.07.2007**
(21) Application number: 05783167.9
(22) Date of filing: 13.09.2005
(51) Int. Cl.: A61B 1/00

(54) **INTRODUCTION-INTO-SUBJECT SYSTEM, RECEIVER, AND INTRODUCTION-INTO-SUBJECT DEVICE**

(30) Priority: 13.09.2004 JP 2004266064
(71) Applicant: Olympus Corporation, Tokyo 151-0072 (JP)
(72) Inventor: MINAI, Tetsuo, 1920919 (JP)
(74) Representative: von Hellfeld, Axel
(86) International application number: PCT/JP2005/016825
(87) International publication number: WO 2006/030772

(57) **Abstract**

A receiving apparatus 3 which is an element of a body insertable system is configured with a receiving unit 6 including receiving antennas 8a to 8d and a reception processing device 9, and a position detecting unit 7 including transmitting antennas 10a to 10d, a first linear magnetic field generator 11a, a second linear magnetic field generator 11b, a diffuse magnetic field generator 12, and a processing device 13, and the receiving unit 6 and the position detecting unit 7 are formed separately and independently of each other. Therefore, when the body insertable system is used for acquisition of intra-subject information which is acquired by the capsule endoscope 2 and position detection of the capsule endoscope 2, both the receiving unit 6 and the position detecting unit 7 are used, whereas when the body insertable system is used only for the acquisition of the intra-subject information, it is possible to use only the receiving unit 6, whereby a burden on the subject at a time of use is restricted to a minimum degree according to the purpose of use while increase in operational cost is suppressed.

## Description

### TECHNICAL FIELD

The present invention relates to a body insertable apparatus which is introduced inside a subject, acquires intra-subject information as information concerning the subject, and transmits radio signals including the acquired intra-subject information, a receiving apparatus which performs reception processing of the radio signals transmitted by the body-insertable apparatus, and a body-insertable system configured with the body-insertable apparatus and the receiving apparatus.

### BACKGROUND ART

In recent years, in a field of endoscope, a swallowable capsule endoscope is proposed. The capsule endoscope is provided with an imaging function and a radio communication function. After being swallowed from a mouth of a subject (human body) for an observation (examination) until naturally discharged, the capsule endoscope travels inside body cavities, for example, internal organs such as a stomach and a small intestine following peristaltic movements, and has a function of sequentially capturing images.

while the capsule endoscope travels inside the body cavities, image data acquired through image capturing by the capsule endoscope inside the body is sequentially transmitted to an outside by radio communication and accumulated in a memory provided outside. By carrying a receiving apparatus provided with a radio communication function and a memory function, the subject can move freely after swallowing the capsule endoscope until discharging the same. After the capsule endoscope is discharged, a doctor or a nurse can make diagnosis by displaying images of the internal organs on a display based on the image data accumulated in the memory (see Patent Document 1, for example).

Further, among conventional capsule endoscope systems, some proposed systems include a mechanism to detect a position of the capsule endoscope in a body cavity. For example, it is possible to generate a magnetic field whose strength has a positional dependency inside the subject to which the capsule endoscope is introduced, and to detect the position of the capsule endoscope in the subject based on the strength of the magnetic field detected by a magnetic field sensor incorporated in the capsule endoscope. Such a capsule endoscope system adopts a structure in which a predetermined coil is arranged outside the subject for generation of a magnetic field, and generates the magnetic field inside the subject by letting a predetermined electric current flow through the coil.

When the position detection mechanism is further provided in the receiving apparatus as described above, various contrivances can be made in the conventional capsule endoscope system, for example, an imaging operation by an imaging mechanism can be started from a time point when the capsule endoscope reaches the small intestine of the subject. Accordingly, there can be advantages, for example, that the image data can be acquired only with respect to a necessary area for the doctor.

Patent Document 1: Japanese Patent Application Laid-Open No. 2003-19111

### DISCLOSURE OF INVENTION

### PROBLEM TO BE SOLVED BY THE INVENTION

However, the conventional capsule endoscope system, having a position detection mechanism with a predetermined size, increases a burden placed on a patient unnecessarily when the position detection is not performed. Such inconveniences will be described in detail below.

In an examination with a capsule endoscope, there is not always a necessity to perform the position detection. For example, in an examination in which images are acquired constantly from an oral cavity to a large intestine of a subject, the examination is carried out without the position detection. In such an examination, the position detection mechanism provided in the receiving apparatus is not necessary, and the subject carries the receiving apparatus provided with the unnecessary position detection mechanism until the examination ends, whereby the burden on the subject increases unnecessarily, which is not desirable.

To alleviate the above inconvenience, it may be possible to prepare the receiving apparatus provided with the position detection mechanism and the receiving apparatus not provided with the position detection mechanism, and to selectively use each of the receiving apparatuses depending on a purpose of an examination. However, when such a structure is adopted, various types of receiving apparatuses become necessary, and costs required for the examination with the capsule endoscope increase, leading to another inconvenience.

The present invention is made in view of the above, and an object is to realize a subject insertable system provided with a body-insertable apparatus such as a capsule endoscope to allow to restrict a burden on the subject at a use of the body insertable system to a minimum degree depending on a purpose of use while suppressing an increase in operational cost.

### MEANS FOR SOLVING PROBLEM

To solve the problems as described above and to achieve the objects, a body insertable system according to claim 1 includes a body insertable apparatus that is introduced inside a subject, acquires intra-subject information as information concerning the subject, and transmits radio signals including the acquired intra-subject information, and a receiving apparatus that performs reception processing of the radio signals transmitted by the body insertable apparatus. The body insertable apparatus includes an intra-subject information acquiring unit that acquires the intra-subject information; a magnetic field sensor that detects a magnetic field in a region where the body insertable apparatus is located; and a radio transmitting unit that transmits radio signals including at least the intra-subject information. The receiving apparatus includes a receiving unit that includes at least a receiving antenna which receives the radio signals transmitted by the body insertable apparatus and a receiving circuit which performs reception processing on the radio signals received by the receiving antenna; and a position detecting unit that includes a magnetic field generator which generates a predetermined magnetic field for position detection in a region where the body insertable apparatus can be present, and a position calculator that calculates a position of the body insertable apparatus based on a result of detection of the magnetic field for position detection acquired by the magnetic field sensor, the position detecting unit being formed separately and independently of the receiving unit.

According to the present invention as set forth in claim 1, since the receiving unit and the position detecting unit are formed separately and independently of each other, the burden on the subject can be minimized according to the purpose of use. Specifically, when the acquisition of the intra-subject information alone is the purpose of use, the position detecting unit can be removed from the receiving apparatus and the receiving unit alone can be used, whereby the burden on the subject can be reduced.

Further, in the body insertable system according to the present invention as set forth in claim 2, the radio transmitting unit transmits radio signals including a result of detection acquired by the magnetic field sensor in addition to the intra-subject information, and the position detecting unit acquires the result of detection acquired by the magnetic field sensor via the receiving unit.

Still further, in the body insertable system according to the present invention as set forth in claim 3, the position detecting unit is arranged in a fixed state relative to the subject at a time of use, and the receiving unit is arranged in a movable state relative to the subject at a time of use.

Still further, a receiving apparatus according to claim 4 performs reception processing of a radio signal transmitted from a predetermined detection target, and includes a receiving unit that includes at least a receiving antenna which receives the radio signal transmitted from the detection target, and a receiving circuit which performs reception processing on the radio signal received by the receiving antenna; and a position detecting unit that includes a magnetic field generator which generates a predetermined magnetic field for position detection in a region where the detection target can be present, and a position calculator which calculates a position of the detection target based on a result of detection of the magnetic field for position detection in the region where the detection target can be present, the position detecting unit being formed separately and independently of the receiving unit.

Still further, a body insertable apparatus according to claim 5 is introduced inside a subject and acquires intra-subject information as information concerning the subject, and includes an intra-subject information acquiring unit that acquires the intra-subject information; a magnetic field sensor that detects a magnetic field in a region where the body insertable apparatus is located; a radio transmitting unit that transmits radio signals including at least the intra-subject information; and a magnetic field detection controller that controls a driven state of the magnetic field sensor.

According to the present invention as set forth in claim 5, the body insertable apparatus can be employed only for an acquisition of the intra-subject information and for both the acquisition of the intra-subject information and position detection utilizing the magnetic field for position detection.

Still further, the body insertable apparatus according to the present invention as set forth in claim 6 further includes a radio receiving unit that receives a radio signal transmitted from an outside, and the magnetic field detection controller controls the driven state of the magnetic field sensor based on a control signal received by the radio receiving unit.

Still further, in the body insertable apparatus according to the present invention as set forth in claim 7, the magnetic field sensor performs a magnetic field detection in a stand-by mode in which a detection interval is longer than in a normal mode when the magnetic field for position detection is not generated in the region where the body insertable apparatus is located, and transits from the stand-by mode to the normal mode when the magnetic field for position detection is detected during the stand-by mode.

### EFFECT OF THE INVENTION

The body insertable system and the receiving apparatus according to the present invention are advantageous in that the burden on the subject can be minimized depending on the purpose of use since the receiving unit and the position detecting unit are formed separately and independently of each other. When the purpose is only to acquire the intra-subject information, the position detecting unit can be removed with respect to the receiving apparatus and the receiving unit alone can be used, whereby there is an advantage that the burden on the subject can be reduced.

Further, the body insertable apparatus according to the present invention is advantageous in that the body insertable apparatus can be used both for the purpose of only acquiring the intra-subject information, and for the purpose of position detection utilizing the intra-subject information and the magnetic field for position detection.

### BRIEF DESCRIPTION OF DRAWINGS

FIG. 1 is a schematic diagram showing an overall structure of a body insertable system according to a first embodiment;
FIG. 2 is a schematic block diagram showing a structure of a capsule endoscope provided in the body insertable system;
FIG. 3 is a schematic block diagram showing a structure of a receiving apparatus provided in the body insertable system;
FIG. 4 is a schematic diagram showing a mode of a first linear magnetic field generated by a first linear magnetic field generator provided in a position detecting unit constituting the receiving apparatus;
FIG. 5 is a schematic diagram showing a structure of a second linear magnetic field generator and a diffuse magnetic field generator provided in the position detecting unit together with a mode of a second linear magnetic field generated by the second linear magnetic field generator;
FIG. 6 is a schematic diagram showing a mode of a diffuse magnetic field generated by the diffuse magnetic field generator;
FIG. 7 is a flowchart for describing an operation of a capsule endoscope;
FIG. 8 is a flowchart for describing an operation of the position detecting unit;
FIG. 9 is a schematic diagram showing relations between reference coordinate axes and target coordinate axes;
FIG. 10 is a schematic diagram showing a mode of use of the second linear magnetic field at a position calculation;
FIG. 11 is a schematic diagram showing a mode of use of the diffuse magnetic field at a position calculation;
FIG. 12 is a schematic block diagram showing a structure of a capsule endoscope provided in a body insertable system according to a second embodiment;
FIG. 13 is a schematic block diagram showing a structure of a receiving apparatus provided in the body insertable system;
FIG. 14 is a flowchart for describing an operation of a position detecting unit constituting a receiving apparatus;
FIG. 15 is a flowchart for describing an operation of a capsule endoscope;
FIG. 16 is a schematic diagram showing an overall structure of a body insertable system according to a third embodiment; and
FIG. 17 is a schematic block diagram showing a structure of a processing device provided in a receiving apparatus constituting the body insertable system.

### EXPLANATIONS OF LETTERS OR NUMERALS

- 1: Subject
- 2, 63: Capsule endoscope
- 3, 70: Receiving apparatus
- 4: Display device
- 5: Portable recording medium
- 6: Receiving unit
- 7, 67, 71: Position detecting unit
- 8a to 8d, 28: Receiving antenna
- 9: Reception processing device
- 10a to 10d, 27: Transmitting antenna
- 11a: First linear magnetic field generator
- 11b: Second linear magnetic field generator
- 12: Diffuse magnetic field generator
- 13, 68, 72: Processing device
- 14: Intra-subject information acquiring unit
- 15, 30, 37: Signal processing unit
- 16: Magnetic field sensor
- 17: Amplifying unit
- 18: A/D converter
- 19: Radio transmitting unit
- 20: Switching unit
- 21: Timing generator
- 22: LED
- 23: LED driving circuit
- 24: CCD
- 25: CCD driving circuit
- 26, 49: Transmitting circuit
- 29, 36: Receiving circuit
- 31, 65: Magnetic field detection controller
- 32: Condenser
- 33: Radio receiving unit
- 34, 56, 58: Coil
- 35: Receiving antenna selector
- 38: Recording unit
- 39, 51: Selection controller
- 41, 44: Input/output interface
- 42, 53: Power supply unit
- 45: Orientation calculator
- 46: Position calculator
- 47: Magnetic-field line orientation database
- 48: Control signal generator
- 50: Transmitting antenna selector
- 52: Magnetic field generation controller
- 54: Transmitting unit
- 57, 59: Electric current source
- 61: Curved surface
- 64: Magnetic field strength calculator
- 73: Earth-magnetism sensor
- 74: Earth-magnetism orientation calculator

### BEST MODE(S) FOR CARRYING OUT THE INVENTION

Exemplary embodiments of the present invention (hereinafter simply referred to as "embodiments"), i.e., a body insertable apparatus, a receiving apparatus, and a body insertable system will be described below. The present invention is not limited to the embodiments. The drawings are merely schematic; it should be noted that relations between thickness and width of each portion and a ratio of thickness of one portion to thickness of another portion may be different from actual ones; and each drawing may include portions with different dimensional relation and different ratio.

### First Embodiment

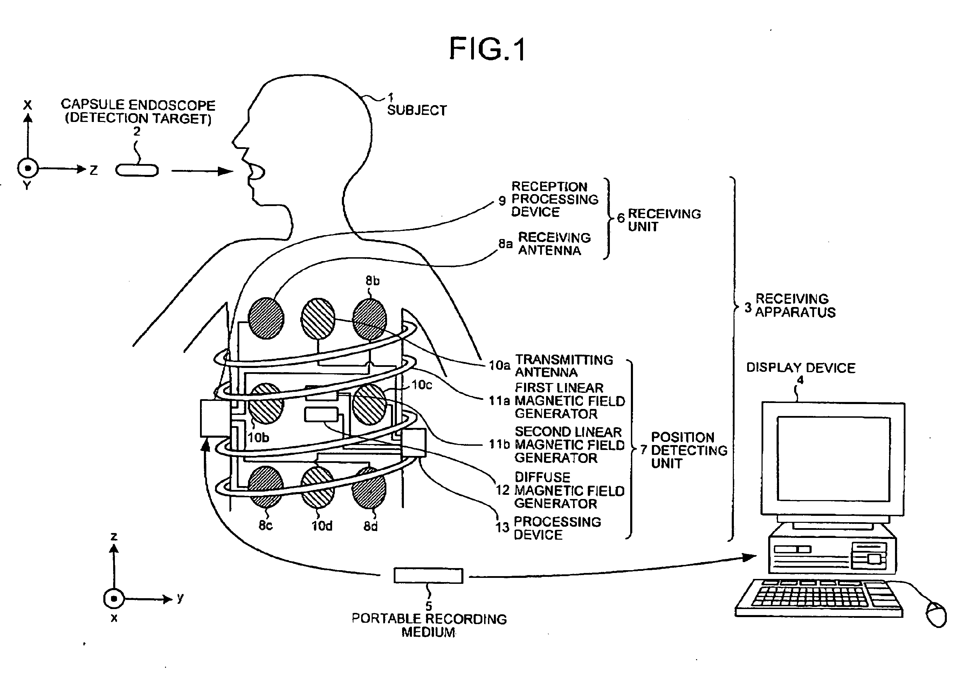

First, a body insertable system according to a first embodiment will be described. FIG. 1 is a schematic diagram showing an overall structure of a body insertable system according to the first embodiment. As shown in FIG. 1, the body insertable system according to the first embodiment includes a capsule endoscope 2 which is introduced inside a subject 1, a receiving apparatus 3 which performs reception processing and the like of radio signals transmitted by the capsule endoscope 2, a display device 4 which displays contents of the radio signals transmitted from the capsule endoscope 2 and received by the receiving apparatus 3, and a portable recording medium 5 which serves for information delivery between the receiving apparatus 3 and the display device 4. Further, as shown in FIG. 1, in the first embodiment, target coordinate axes which consist of an X-axis, a Y-axis, and a Z-axis, and are fixed relative to the capsule endoscope 2, and reference coordinate axes which consist of an x-axis, a y-axis, and a z-axis, are set irrespective of a movement of the capsule endoscope 2, and are fixed particularly relative to the subject 1 are set. A position detecting unit 7 described later is made to detect positional relations of the target coordinate axes relative to the reference coordinate axes.

The display device 4 serves to display intra-subject images or the like acquired through image capturing by the capsule endoscope 2 and received by the receiving apparatus 3, and is configured like a workstation or the like that displays images based on data acquired from the portable recording medium 5. Specifically, the display device 4 may be configured so as to directly display images or the like as in a CRT display and a liquid crystal display, or alternatively, may be configured so as to output images or the like to other media as in a printer.

The portable recording medium 5 is attachable/detachable to/from a reception processing device 9 described later and the display device 4, and is configured so as to allow for output and recording of information when attached to the above two devices. Specifically, the portable recording medium 5, while the capsule endoscope 2 travels through body cavities of the subject 1, is attached to the reception processing device 9 and stores intra-subject images and positional relations of the target coordinate axes relative to the reference coordinate axes. The portable recording medium 5 is configured so as to be taken out from the reception processing device 9 and attached to the display device 4, after the capsule endoscope 2 is discharged from the subject 1, so that the recorded data is read out by the display device 4. When the data delivery between the reception processing device 9 and the display device 4 is performed with the portable recording medium 5 such as a Compact Flash (registered trademark) memory or the like, dissimilar to a system in which the reception processing device 9 and the display device 4 are connected with a cable, the subject 1 can move freely even while the capsule endoscope 2 travels inside the subject 1.

Next, the capsule endoscope 2 will be described. The capsule endoscope 2 functions as an example of a detection target and a body insertable apparatus according to the present invention. Specifically, the capsule endoscope 2 has functions of being introduced inside the subject 1, acquiring intra-subject information while traveling inside the subject 1, and transmitting radio signals including the acquired intra-subject information to an outside. Further, the capsule endoscope 2 has a magnetic field detection function for detecting positional relation described later and at the same time is configured so as to receive driving power from the outside, and specifically, the capsule endoscope 2 has functions of receiving radio signals transmitted from the outside and reproducing the driving power from the received radio signals.

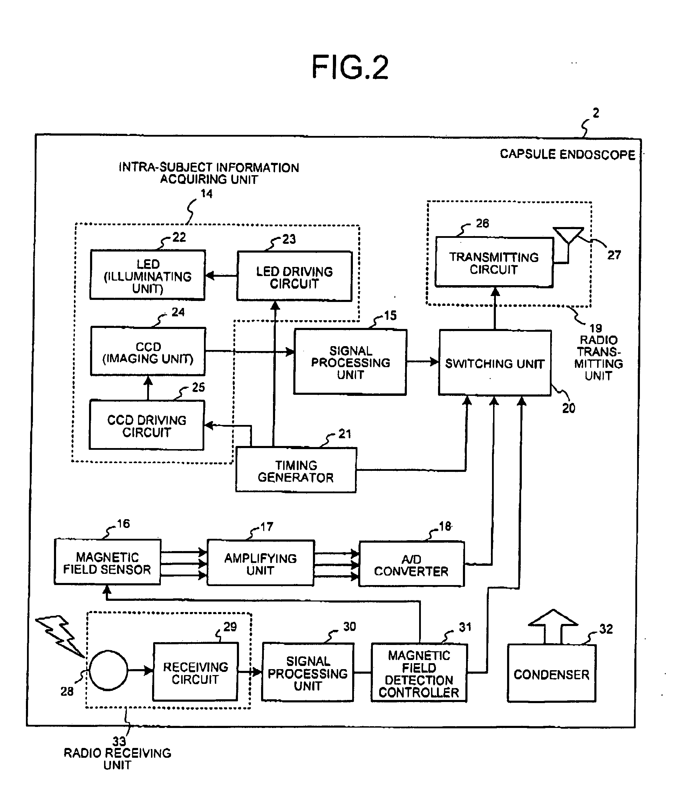

FIG. 2 is a block diagram showing a structure of the capsule endoscope 2. As shown in FIG. 2, the capsule endoscope 2 includes an intra-subject information acquiring unit 14 which acquires intra-subject information as a mechanism for acquiring the intra-subject information, and a signal processing unit 15 which performs predetermined processing on the acquired intra-subject information. Further, the capsule endoscope 2 includes a magnetic field sensor 16 which detects a magnetic field as a magnetic field detection mechanism and outputs electric signals corresponding to the detected magnetic field, an amplifying unit 17 which serves for amplifying the supplied electric signals, and an A/D converter 18 which converts the electric signals output from the amplifying unit 17 into digital signals.

The intra-subject information acquiring unit 14 serves to acquire intra-subject information, which is, in the first embodiment, an intra-subject image that is image data of the inside of the subject 1. Specifically, the intra-subject information acquiring unit 14 includes an LED 22 which functions as an illuminating unit, an LED driving circuit 23 which controls driving of the LED 22, a CCD 24 which functions as an imaging unit that captures images of at least a portion of an area illuminated by the LED 22, and a CCD driving circuit 25 which controls a driven state of the CCD 24. Here, as specific structures of the illuminating unit and the imaging unit, the use of the LED and the CCD is not essential, and a CMOS or the like can be employed as the imaging unit.

The magnetic field sensor 16 serves to detect an orientation and a strength of a magnetic field generated in a region where the capsule endoscope 2 is present. Specifically, the magnetic field sensor 16 is formed with an MI (Magneto Impedance) sensor, for example. The MI sensor is configured, for example, with a FeCoSiB amorphous wire as a magneto-sensitive medium, and detects the strength of the magnetic field by utilizing MI effect, i.e., the effect that magnetic impedance of the magneto-sensitive medium exhibits significant fluctuation attributable to an external magnetic field when a high-frequency electric current is conducted to the magneto-sensitive medium. The magnetic field sensor 16 may be configured with an element other than the MI sensor, for example, with an MRE (Magneto Resistive Effect) element, and a GMR (Giant Magneto Resistive Effect) magnetic sensor.

As shown also in FIG. 1, in the first embodiment, as coordinate axes of the capsule endoscope 2 as the detection target, target coordinate axes defined by the X-axis, the Y-axis, and the Z-axis are set. Corresponding to the target coordinate axes, the magnetic field sensor 16 has functions of detecting an X-direction component, an Y-direction component, and a Z-direction component of the strength of a magnetic field generated in a region where the capsule endoscope 2 is present, and outputting an electric signal corresponding to the strength of the magnetic field in each direction. The magnetic-field strength components in the target coordinate axes as detected by the magnetic field sensor 16 are transmitted to the receiving apparatus 3 via a radio transmitting unit 19 described later, and the receiving apparatus 3 calculates positional relations between the target coordinate axes and the reference coordinate axes based on the values of the magnetic field components detected by the magnetic field sensor 16.

Further, the capsule endoscope 2 includes a radio transmitting unit 19 which includes a transmitting circuit 26 and a transmitting antenna 27 and serves to perform radio transmission to the outside, and a switching unit 20 which appropriately switches a signal output to the radio transmitting unit 19 between a signal output from the signal processing unit 15 and a signal output from the A/D converter 18. Further, the capsule endoscope 2 includes a timing generator 21 which serves to synchronize driving timings of the intra-subject information acquiring unit 14, the signal processing unit 15, and the switching unit 20.

Further, the capsule endoscope 2 has a function of controlling a driven state of the magnetic field sensor 16 and the like based on radio signals transmitted from the outside. Specifically, the capsule endoscope 2 includes a radio receiving unit 33 which receives radio signals transmitted from the position detecting unit 7 described later, a signal processing unit 30 which extracts predetermined control signals by performing predetermined processing on the received radio signals, and a magnetic field detection controller 31 which controls driven states of the magnetic field sensor 16 and the switching unit 20 based on the control signals.

The radio receiving unit 33 includes a receiving antenna 28, and a receiving circuit 29 which performs predetermined processing such as demodulation processing on the radio signals received via the receiving antenna 28. Further, the magnetic field detection controller 31 has a function of controlling a driven state of the magnetic field sensor 16 and the like according to contents of the control signals, and in a most simple structure, the magnetic field detection controller 31 controls so as to stop driving of the magnetic field sensor 16 and the like in a state in which no control signals are input, and to drive the magnetic field sensor 16 and the like in response to the input of the control signal.

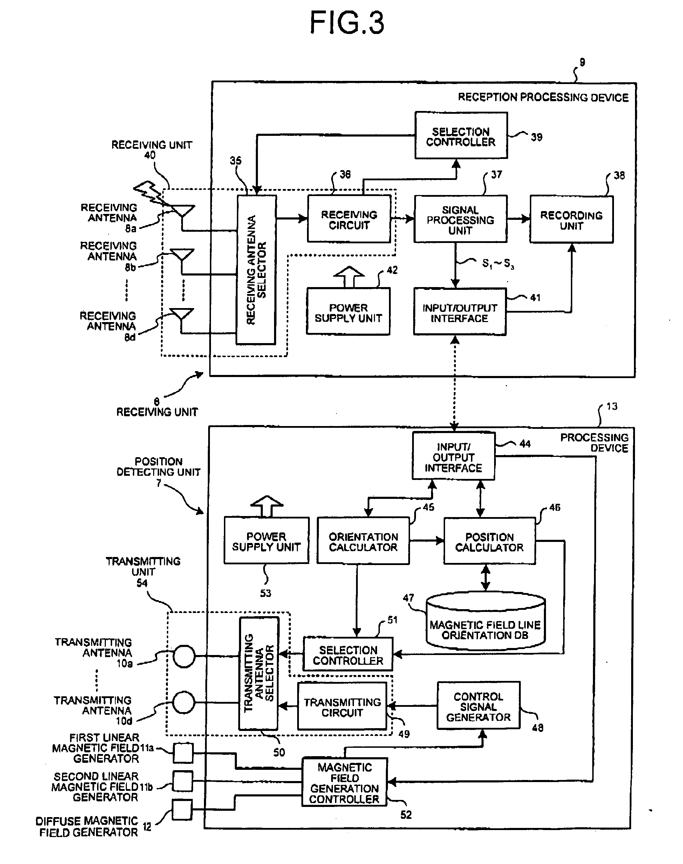

Next, the receiving apparatus 3 will be described. As shown in FIG. 1, the receiving apparatus 3 is configured with a receiving unit 6 and the position detecting unit 7 that are formed separately and independently of each other, and the receiving apparatus 3 is configured so as to operate not only in a state where the receiving unit 6 and the position detecting unit 7 are combined, but also only with the receiving unit 6. FIG. 3 is a schematic block diagram showing an overall structure of the receiving apparatus 3. In the following, a structure of the receiving unit 6 will be described first, followed by a description on a structure of the position detecting unit 7.

The receiving unit 6 includes, as shown in FIGS. 1 and 3, receiving antennas 8a to 8d that serve to receive the radio signals transmitted from the capsule endoscope 2, and the reception processing device 9 which performs reception processing and the like on the radio signals received via one of the receiving antennas 8a to 8d.

The receiving antennas 8a to 8d serve to receive radio signals transmitted from the radio transmitting unit 19 provided in the capsule endoscope 2. Specifically, the receiving antennas 8a to 8d are formed with a loop antenna or the like, and used while being arranged on an outer surface of the subject 1.

The reception processing device 9 serves to perform reception processing and the like on the radio signals received via one of the receiving antennas 8a to 8d. Specifically, the reception processing device 9 includes a receiving antenna selector 35 which selects one of the receiving antennas 8a to 8d, a receiving circuit 36 which extracts an original signal included in the radio signal by performing demodulation processing and the like on the radio signal received via the selected receiving antenna, and a signal processing unit 37 which reconfigures an image signal and the like by processing the extracted original signal.

Specifically, the signal processing unit 37 has functions of reconfiguring magnetic field signals S₁ to S₃ and an image signal S₄ based on the extracted original signal, and outputting the reconfigured signals to suitable elements, respectively. Here, the magnetic field signals S₁ to S₃ are magnetic field signals corresponding to a first linear magnetic field, a second linear magnetic field, and a diffuse magnetic field, respectively, detected by the magnetic field sensor 16, and are reconfigured when the receiving unit 6 and the position detecting unit 7 are used in a combined state as described later. Further, the image signal S₄ corresponds to an intra-subject image acquired by the intra-subject information acquiring unit 14. As to specific forms of the magnetic field signals S₁ to S₃, the magnetic field signals S₁ to S₃ are represented by direction vectors corresponding to the detected magnetic field strength in the target coordinate axes fixed relative to the capsule endoscope 2, and include information related with an advance direction of the magnetic field in the target coordinate axes and the magnetic field strength.

Further, the reception processing device 9 includes a recording unit 38 which has a function of recording the image signals S₄ and the like reconfigured by the signal processing unit 37 into the portable recording medium 5, a selection controller 39 which controls a manner of antenna selection by the receiving antenna selector 35 based on the magnetic field strength signal and the like output from the receiving circuit 36, an input/output interface 41 which serves for input/output of information into/from the position detecting unit 7, and a power supply unit 42 which supplies driving power to elements provided in the reception processing device 9.

The recording unit 38 has a function of recording input data into the portable recording medium 5. The recording unit 38 is configured so as to receive inputs of position information of the capsule endoscope 2 as calculated by the position detecting unit 7 and input via the input/output interface 41, in addition to the aforementioned image signals S₄.

The selective controller 39 serves to select a receiving antenna which is appropriate for the reception from the receiving antennas 8a to 8d. Specifically, the selection controller 39 has a function of determining the receiving antenna 8 with a highest received signal strength based on information (RSSI (Received Signal Strength Indicator), for example) related to received signal strength and generated by the receiving circuit 36, and controlling the receiving antenna selector 35 so as to select the determined receiving antenna 8.

The input/output interface 41 serves for information delivery to/from the position detecting unit 7. Specifically, in the first embodiment, the input/output interface 41 at least outputs the magnetic field signals S₁ to S₃ to the position detecting unit 7, and inputs information concerning the position of the capsule endoscope 2 from the position detecting unit 7 side. As a specific structure of the input/output interface 41, any structure can be adopted as far as the structure allows for the input/output of information. For example, the input/output interface 41 may be configured so as to be connected by a cable with an input/output interface 44 (described later) provided in the position detecting unit 7, or alternatively, may be configured for a wireless connection.

Next, the position detecting unit 7 will be described. As shown in FIGS. 1 and 3, the position detecting unit 7 includes transmitting antennas 10a to 10d for transmitting the radio signals to the capsule endoscope 2, a first linear magnetic field generator 11a, a second linear magnetic field generator 11b, and a diffuse magnetic field generator 12 that generate the first linear magnetic field, the second magnetic field, and the diffuse magnetic field, respectively, as a magnetic field for position detection, and a processing device 13 that performs predetermined information processing. In the following, a structure of the processing device 13 will be described first, followed by description on the first linear magnetic field generator 11a, the second linear magnetic field generator 11b, and the diffuse magnetic field generator 12.

The processing device 13 includes, as shown in FIG. 3, the input/output interface 44 which serves for information delivery to/from the input/output interface 41 provided in the receiving unit 6, an orientation calculator 45 which calculates an orientation of the target coordinate axes relative to the reference coordinate axes based on the magnetic field signals S₁ and S₂ corresponding to the detected strength of the first linear magnetic field and the second linear magnetic field among the information output from the receiving unit 6, a position calculator 46 which calculates a position of the capsule endoscope 2 using the magnetic field signal S₃ corresponding to the detected strength of the diffuse magnetic field, the magnetic field signal S₂, and the result of calculation by the orientation calculator 45, and a magnetic-field line orientation database 47 which records correspondence between an advance direction and a position of the magnetic field line constituting the diffuse magnetic field at the position calculation by the position calculator 46. The orientation calculation and the position calculation by the above listed elements will be described later in detail.

Further, the processing device 13 has functions of radio transmitting the control signals to the capsule endoscope 2 and controlling driving of the first linear magnetic field generator 11a and the like. Specifically, the processing device 13 includes a control signal generator 48 which generates the control signals, a transmitting circuit 49 which generates predetermined radio signals based on radio signals including the generated control signals, a transmitting antenna selector 50 which selects an antenna to transmit the generated radio signals from the transmitting antennas 10a to 10d, and a selection controller 51 which controls a manner of selection of the transmitting antenna. Further, the processing device 13 includes a magnetic field generation controller 52 which controls driven states of the first linear magnetic field generator 11a, the second linear magnetic field generator 11b, the diffuse magnetic field generator 12, and the control signal generator 48.

The control signal generator 48 has a function of generating control signals to be supplied to the magnetic field detection controller 31 provided in the capsule endoscope 2. As a content of the control signal any content can be employed, for example, if the magnetic field detection controller 31 has a function of driving the magnetic field sensor 16 and the like on receiving some signals, the control signal may consists of a single pulse, for example.

The selection controller 51 serves to determine a manner of selection of the transmitting antenna 10 to be used for transmission of the radio signal including the control signal. Specifically, the selection controller 51 has a function of determining the transmitting antenna 10, which can most efficiently transmit the radio signal to the capsule endoscope 2, based on the results of calculation by the orientation calculator 45 and the position calculator 46. In particular, the selection controller 51 grasps a position of the receiving antenna 28 provided in the capsule endoscope 2 on the target coordinate axes in advance, and acquires the positional relations between the target coordinate axes and the reference coordinate axes according to the results of calculation by the orientation calculator 45 and the position calculator 46. The selection controller 51 has functions of grasping positional relations between the transmitting antennas 10a to 10d and the receiving antenna 28 provided in the capsule endoscope 2 based on the acquired positional relations, determining the transmitting antenna 10 which is most appropriate for the transmission, and controlling the transmitting antenna selector 50 so as to select the determined antenna.

The magnetic field generation controller 52 serves to control a driven state of the magnetic field generators such as the first linear magnetic field generator 11a, as well as a driven state of the control signal generator 48. Specifically, the magnetic field generation controller 52 has functions of controlling to stop the driving of the first linear magnetic field generator 11a and the like when the position detecting unit 7 is not used in combination with the receiving unit 6, and to start the driving of the first linear magnetic field generator 11a and the like when the position detecting unit 7 is used in combination with the receiving unit 6. Specifically, in the first embodiment, the magnetic field generation controller 52 has a function of detecting that the input/output of the information to/from the input/output interface 44 from/to the input/output interface 41 provided in the receiving unit 6 becomes possible. The magnetic field generation controller 52 has functions of determining that the position detecting unit 7 is combined with the receiving unit 6 when the information input/output is allowed, and starting the driving of the first linear magnetic field generator 11a and the like.

Further, the processing device 13 has a mechanism for supplying the driving power to the elements described above. Specifically, the processing device 13 has a power supply unit 53 and is configured so as to supply power stored in the power supply unit 53 to each element.

Next, the first linear magnetic field generator 11a, the second linear magnetic field generator 11b, and the diffuse magnetic field generator 12, that are further elements in the position detecting unit 7 will be described. The first linear magnetic field generator 11a, the second linear magnetic field generator 11b, and the diffuse magnetic field generator 12 function as an example of the magnetic field generator recited in the appended claims, and the first linear magnetic field, the second linear magnetic field, and the diffuse magnetic field generated by the respective magnetic field generators function as examples of the magnetic field for position detection recited in the appended claims.

The first linear magnetic field generator 11a serves to generate a linear magnetic field in a predetermined direction inside the subject 1. Here, "linear magnetic field" means a magnetic field consisting of magnetic field components of substantially only one direction within at least a predetermined space region, i.e., a space region in which the capsule endoscope 2 inside the subject 1 can be present in the first embodiment. Specifically, the first linear magnetic field generator 11a includes, as shown in FIG. 1, a coil which is formed so as to cover a torso of the subject 1, and an electric current source (not shown) which supplies predetermined electric currents to the coil, and the first linear magnetic field generator 11a has a function of generating a linear magnetic field in a space region inside the subject 1 by making the predetermined electric currents flow through the coil. Here, as an advance direction of the first linear magnetic field, any direction can be selected, however, in the first embodiment, the first linear magnetic field is set as a linear magnetic field which advances in the z-axis direction on the reference coordinate axes fixed relative to the subject 1.

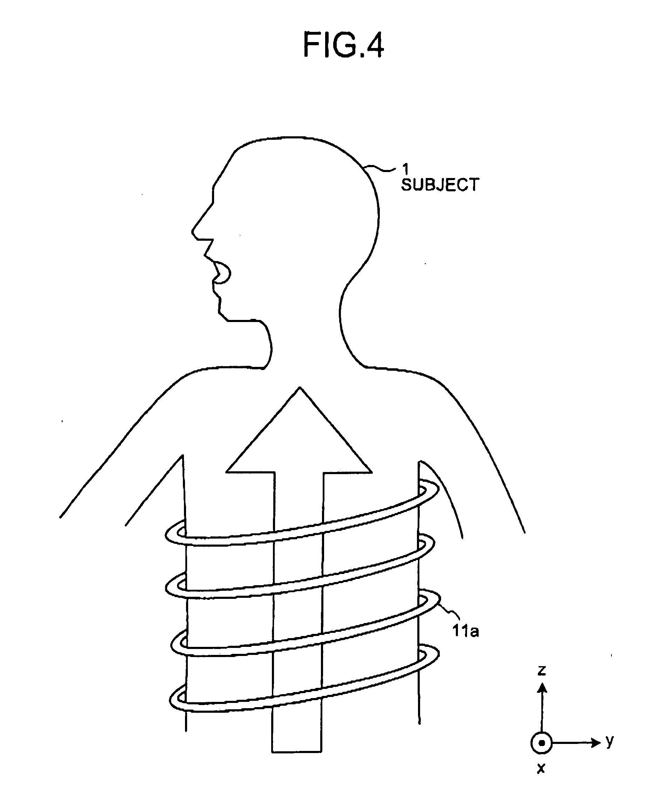

FIG. 4 is a schematic diagram showing the first linear magnetic field generated by the first linear magnetic field generator 11a. As shown in FIG. 4, the coil constituting the first linear magnetic field generator 11a is formed so as to run around the torso of the subject 1 and is configured so as to extend in the z-axis direction on the reference coordinate axes. Therefore, in the first linear magnetic field generated by the first linear magnetic field generator 11a inside the subject 1, a magnetic field line is formed so as to advance in the z-axis direction on the reference coordinate axes, as shown in FIG. 4.

Next, the second linear magnetic field generator 11b and the diffuse magnetic field generator 12 will be described. The second linear magnetic field generator 11b and the diffuse magnetic field generator 12 function as examples of the magnetic field generator as recited in the appended claims, and the second linear magnetic field and the diffuse magnetic field generated by the respective magnetic field generators function as examples of the magnetic field for position detection as recited in the appended claims. In the following description, the second linear magnetic field generator 11b will be specifically described as an example of the magnetic field generator, although as is apparent from the description, the description applies similarly to the diffuse magnetic field generator 12 as an example of the magnetic field generator.

The second linear magnetic field generator 11b serves to generate the second linear magnetic field which is a linear magnetic field advances in a different direction from the advance direction of the first linear magnetic field. Further, the diffuse magnetic field generator 12, being different from the first linear magnetic field generator 11a and the second linear magnetic field generator 11b, serves to generate a diffuse magnetic field whose magnetic field direction has a positional dependency, i.e., in the first embodiment, a magnetic field which diffuses as distanced from the diffuse magnetic field generator 12.

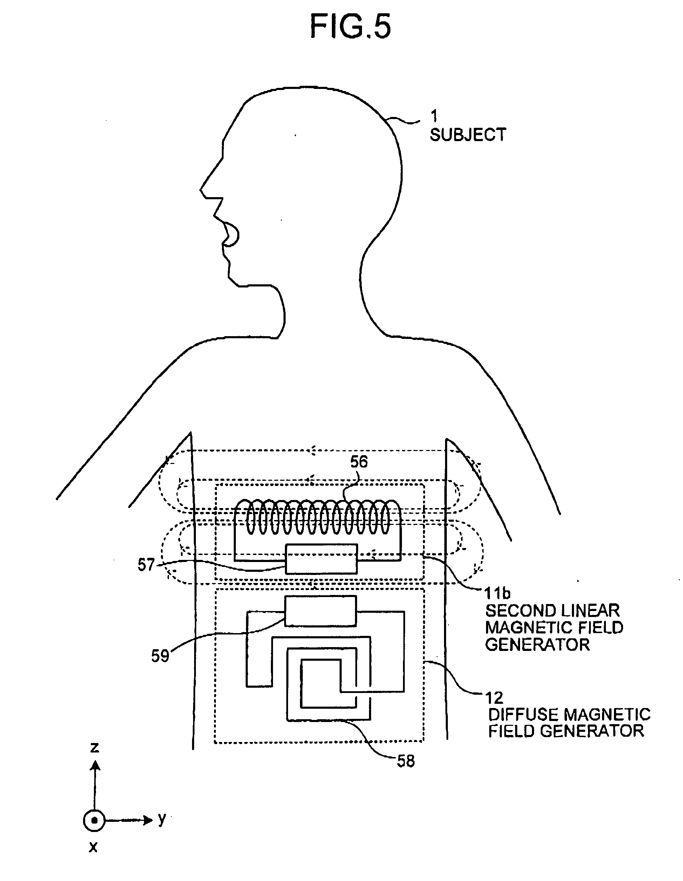

FIG. 5 is a schematic diagram showing a structure of the second linear magnetic field generator 11b and the diffuse magnetic field generator 12, and also showing a mode of the second linear magnetic field generated by the second linear magnetic field generator 11b. As shown in FIG. 5, the second linear magnetic field generator 11b extends in the y-axis direction on the reference coordinate axes, and includes a coil 56 which is formed so that a coil section is parallel to xz-plane, and an electric current source 57 which serves to supply electric currents to the coil 56. Hence, the second linear magnetic field generated by the coil 56 is formed as a linear magnetic field at least inside the subject 1 as shown in FIG. 5, and has a property that the strength thereof decreases according to the distance from the coil 56, in other words, the second linear magnetic field has a positional dependency with respect to the strength.

Further, the diffuse magnetic field generator 12 includes a coil 58, and an electric current source 59 which serves to supply electric currents to the coil 58. Here, the coil 56 is arranged so as to generate a magnetic field whose advance direction is set to a predetermined direction, and in the first embodiment, the coil 56 is arranged so that the advance direction of the linear magnetic field generated by the coil 56 is aligned with the y-axis direction on the reference coordinate axes. Further, the coil 58 is fixed at a position where the coil 58 generates the diffuse magnetic field with the same magnetic field direction as that stored in the magnetic-field line orientation database 47.

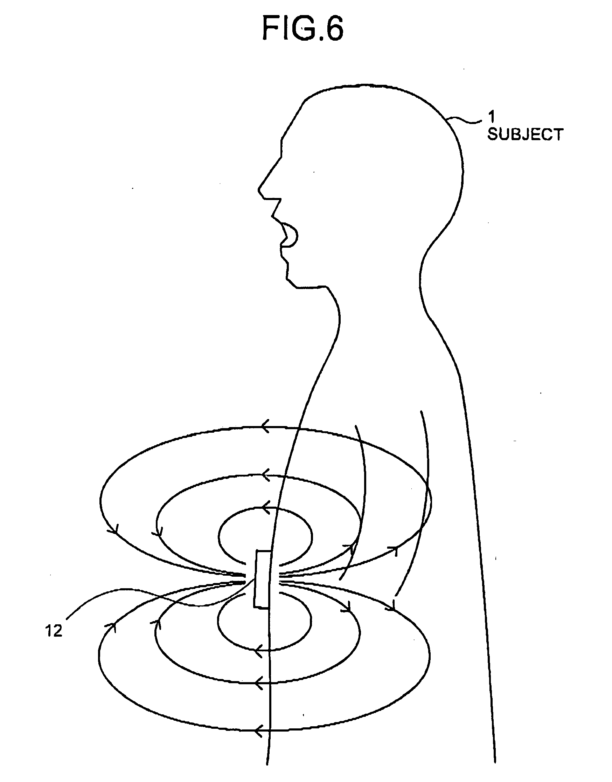

FIG. 6 is a schematic diagram showing a form of the diffuse magnetic field generated by the diffuse magnetic field generator 12. As shown in FIG. 6, the coil 58 provided in the diffuse magnetic field generator 12 is formed in a spiral shape on a surface of the subject 1, and the diffuse magnetic field generated by the diffuse magnetic field generator 12 is formed so that the magnetic field lines are radially diffused once as shown in FIG. 6 and return back to the coil 58 again within the magnetic field generated by the coil 58 (not shown in FIG. 6).

Next, an operation of the body insertable system according to the first embodiment will be described. In the first embodiment, the receiving apparatus 3 is configured with the receiving unit 6 and the position detecting unit 7, and as to the mode of use, the receiving unit 6 operates alone in one mode of use and the receiving unit 6 and the position detecting unit 7 operate in a combined state in another mode of use.

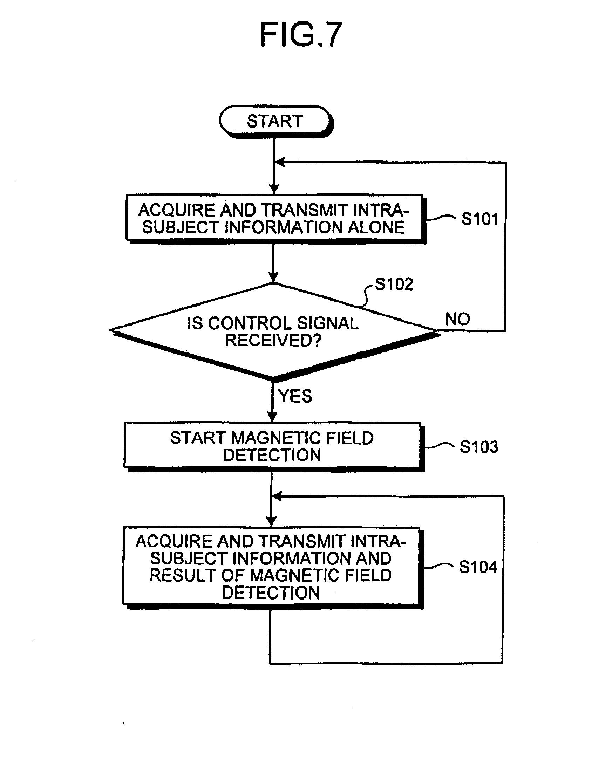

FIG. 7 is a flowchart for describing an operation of the capsule endoscope 2 provided in the body insertable system. The capsule endoscope 2, after being introduced inside the subject 1, acquires only the intra-subject information, and transmits radio signals including the intra-subject information (step S101). At this point, the magnetic field detection controller 31 controls the magnetic field sensor 16 to stop driving, and controls the switching unit 20 so that only the intra-subject information (image data in the first embodiment) output from the signal processing unit 15 is output to the transmitting circuit 26.

The magnetic field detection controller 31 determines whether the radio receiving unit 33 receives the control signals from the position detecting unit 7 or not (step S102), and when the radio receiving unit 33 receives the control signals (Yes in step S102), controls the magnetic field sensor 16 to start the magnetic field detection (step S103), then, the intra-subject information acquiring unit 14 acquires the intra-subject information and at the same time the magnetic field sensor 16 performs the magnetic field detection, and then, the acquired intra-subject information and the result of magnetic field detection are transmitted via the radio transmitting unit 19 (step S104).

When the radio receiving unit 33 does not receive the control signals (No in step S102), the operations in step S101 and S102 are repeated. Time when the radio receiving unit 33 does not receive the control signals means a time when the receiving unit 6 is used alone without being combined with the position detecting unit 7 as described later, and at such a time, the capsule endoscope 2 repeats the operation of step S101.

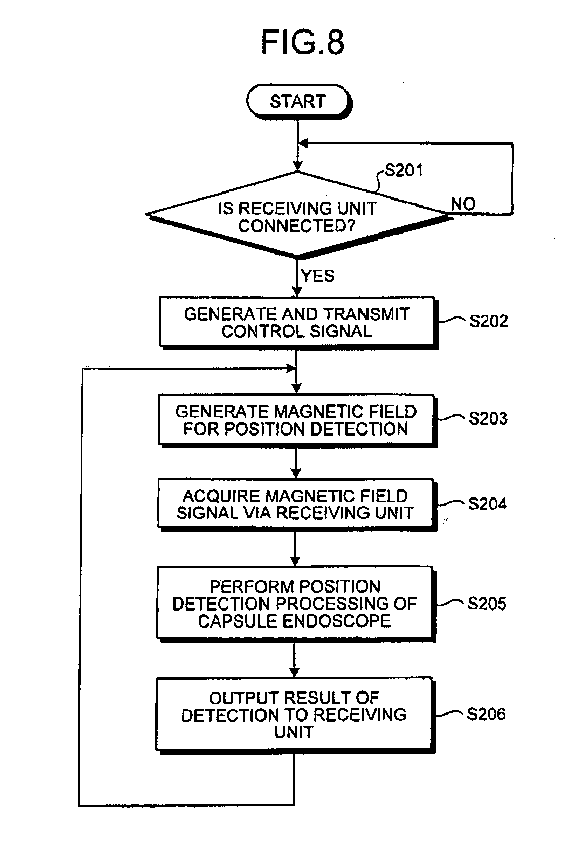

Next, an operation of the receiving apparatus 3 will be described. FIG. 8 is a flowchart showing an operation of the position detecting unit 7 provided in the receiving apparatus 3. Since the receiving unit 6 performs processing which is same as processing in the conventional unit, i.e., processing such as reception processing of the radio signals transmitted from the capsule endoscope 2, regardless of whether the receiving unit 6 is combined with the position detecting unit 7 or not, only an operation of the position detecting unit 7 will be described below.

First, the position detecting unit 7 determines whether the receiving unit 6 is connected thereto or not by the magnetic field generation controller 52 (step S201).
In step S201, the "connection" means that the information delivery through the input/output interfaces 41 and 44 is possible, and the magnetic field generation controller 52 determines by detecting the presence/absence of such a state. When there is no connection (No in step S201), the step S201 is repeatedly performed, whereas when the receiving unit 6 is connected (Yes in step S201), the magnetic field generation controller 52 instructs the control signal generator 48 to generate the control signals, and the generated control signals are radio transmitted via the transmitting unit 54 (step S202). Further, the magnetic field generation controller 52 controls the first linear magnetic field generator 11a and the like so as to start driving, and the first linear magnetic field generator 11a and the like generate predetermined magnetic fields for position detection (step S203). The capsule endoscope 2, by receiving the control signals transmitted in step S202, starts the detection of the magnetic fields for position detection, and transmits radio signals including the result of detection. On the other hand, the position detecting unit 7 acquires the magnetic field signal included in the transmitted radio signals via the receiving unit 6 (step S204), performs position detection processing of the capsule endoscope 2 based on the acquired magnetic field signals (step S205), and outputs the detected position to the receiving unit 6 (step S206). Thereafter, through the repetition of the operations in step S203 to step S206, positions of the capsule endoscope 2 at various times are detected.

Among the processing performed by the position detecting unit 7, the position detection processing in step S205 will be described below. In the body insertable system according to the first embodiment, the structure is made so that the position relations between the reference coordinate axes fixed relative to the subject 1 and the target coordinate axes fixed relative to the capsule endoscope 2 are calculated, and specifically, after the orientations of the target coordinate axes relative to the reference coordinate axes are calculated, the position of an origin of the target coordinate axes on the reference coordinate axes, i.e., the position of the capsule endoscope 2 inside the subject 1 is calculated based on the calculated orientation. Therefore, in the following, an orientation calculation mechanism will be first described, followed by the description on the position calculation mechanism using the calculated orientation. Needless to say, however, devices to which the present invention can be applied are not limited to systems including such position detection mechanism.

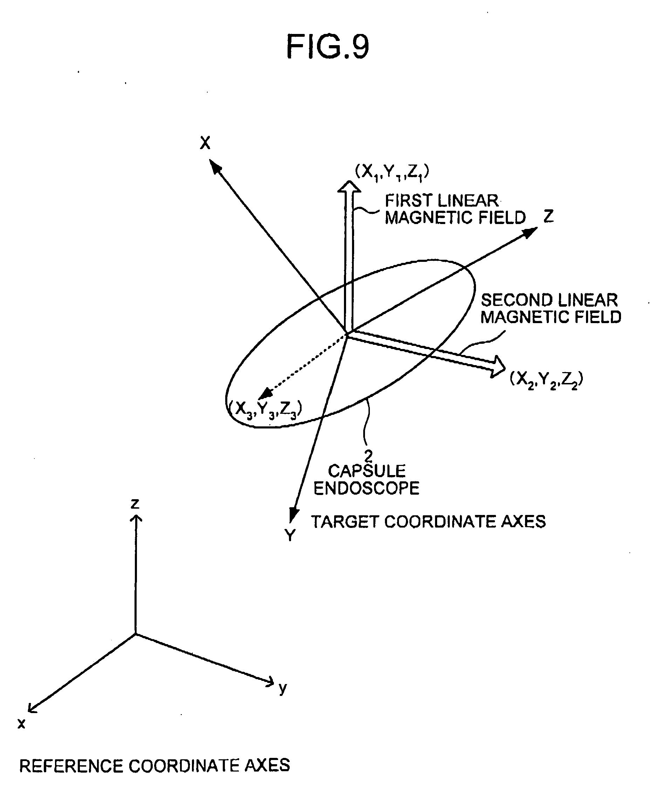

The orientation calculation mechanism of the orientation calculator 45 will be described. FIG. 9 is a schematic diagram showing relations between the reference coordinate axes and the target coordinate axes during the travel of the capsule endoscope 2 inside the subject 1. As described earlier, the capsule endoscope 2 travels along a passage inside the subject 1 and is rotated by a predetermined angle around an axis which extends in an advance direction. Therefore, the target coordinate axes fixed relative to the capsule endoscope 2 is displaced in orientation as shown in FIG. 9 relative to the reference coordinate axes fixed relative to the subject 1.

On the other hand, the first linear magnetic field generator 11a and the second linear magnetic field generator 11b are fixed relative to the subject 1. Therefore, the first linear magnetic field and the second linear magnetic field generated respectively by the first linear magnetic field generator 11a and the second linear magnetic field generator 11b advance in predetermined directions, respectively, with respect to the reference coordinate axes, and specifically, the first linear magnetic field advances in the z-axis direction on the reference coordinate axes and the second linear magnetic field advances in the y-axis direction on the reference coordinate axes.

The orientation calculation in the first embodiment is performed with the use of the first linear magnetic field and the second linear magnetic field. Specifically, the magnetic field sensor 16 provided in the capsule endoscope 2 detects the advance directions of the first linear magnetic field and the second linear magnetic field that are supplied in a time-sharing manner. The magnetic field sensor 16 is configured so as to detect the magnetic field components in the X-axis direction, the Y-axis direction, and the Z-axis direction on the target coordinate axes, and information concerning the detected advance direction of the first linear magnetic field and the second linear magnetic field on the target coordinate axes is transmitted to the receiving apparatus 3 via the radio transmitting unit 19.

The radio signals transmitted by the capsule endoscope 2 are output as the magnetic field signals S₁ and S₂ after processing in the signal processing unit 37 and the like. For example, in an example of FIG. 9, the magnetic field signal S₁ includes information concerning a coordinate (X₁,Y₁,Z₁) as the advance direction of the first linear magnetic field, and the magnetic field signal S₂ includes information concerning a coordinate (X₂,Y₂,Z₂) as the advance direction of the second linear magnetic field. On the other hand, the orientation calculator 45 performs calculation of the orientation of the target coordinate axes relative to the reference coordinate axes in response to the inputs of the magnetic field signals S₁ and S₂. Specifically, the orientation calculator 45 grasps a coordinate (X₃,Y₃,X₃) on the target coordinate axes whose inner products with (X₁,Y₁,Z₁) and (X₂,Y₂,Z₂) are both zero as a coordinate corresponding to the z-axis direction on the reference coordinate axes. Then, the orientation calculator 45 performs predetermined coordinate transformation processing based on the correspondence described above, calculates the coordinates on the target coordinate axes corresponding to the X-axis, the Y-axis, and the Z-axis on the target coordinate axes, and outputs the calculated coordinates as orientation information. The above is the orientation calculation mechanism of the orientation calculator 45.

Next, the position calculation mechanism by the position calculator 46 of the capsule endoscope 2 will be described. The position calculator 46 is configured so as to receive inputs of the magnetic field signals S₂ and S₃ from the signal processing unit 37, to receive an input of the orientation information from the orientation calculator 45, and to receive information stored in the magnetic-field line orientation database 47. The position calculator 46 performs the position calculation of the capsule endoscope 2 based on the supplied information as described below.

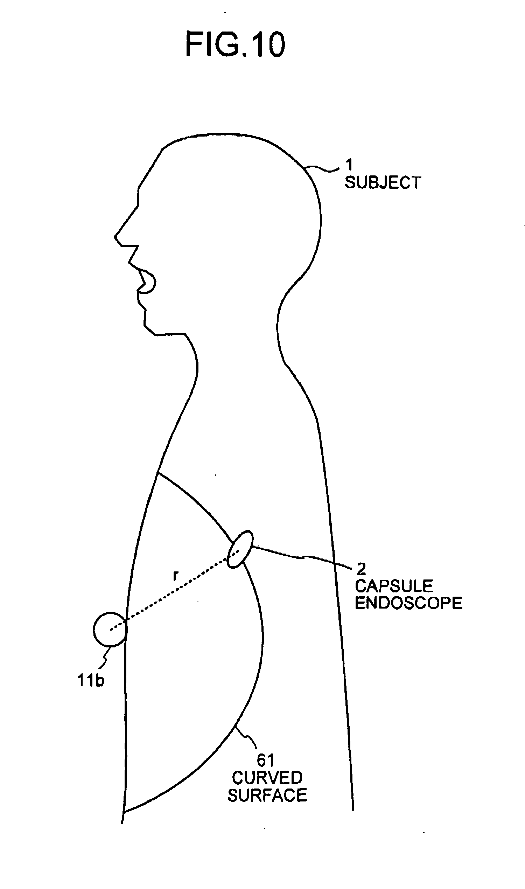

The position calculator 46 calculates a distance between the second linear magnetic field generator 11b and the capsule endoscope 2 using the magnetic field signal S₂. The magnetic field signal S₂ corresponds to the result of detection of the second linear magnetic field in a region where the capsule endoscope 2 is present, and the second linear magnetic field has a property that the strength thereof decreases as distance from the second linear magnetic field generator 11b increases, due to the arrangement of the second linear magnetic field generator 11b outside the subject 1. The position calculator 46, utilizing the above property, compares the strength (which can be found based on the electric current value which flows through the second linear magnetic field generator 11b) of the second linear magnetic field near the second linear magnetic field generator 11b and the strength, which can be found from the magnetic field signal S₂, of the second linear magnetic field in the region where the capsule endoscope 2 is present, and calculates a distance r between the second linear magnetic field generator 11b and the capsule endoscope 2. As a result of calculation of the distance r, it becomes clear that the capsule endoscope 2 is present on a curved surface 61 which is a collection of points distance r away from the second linear magnetic field generator 11b as shown in FIG. 10.

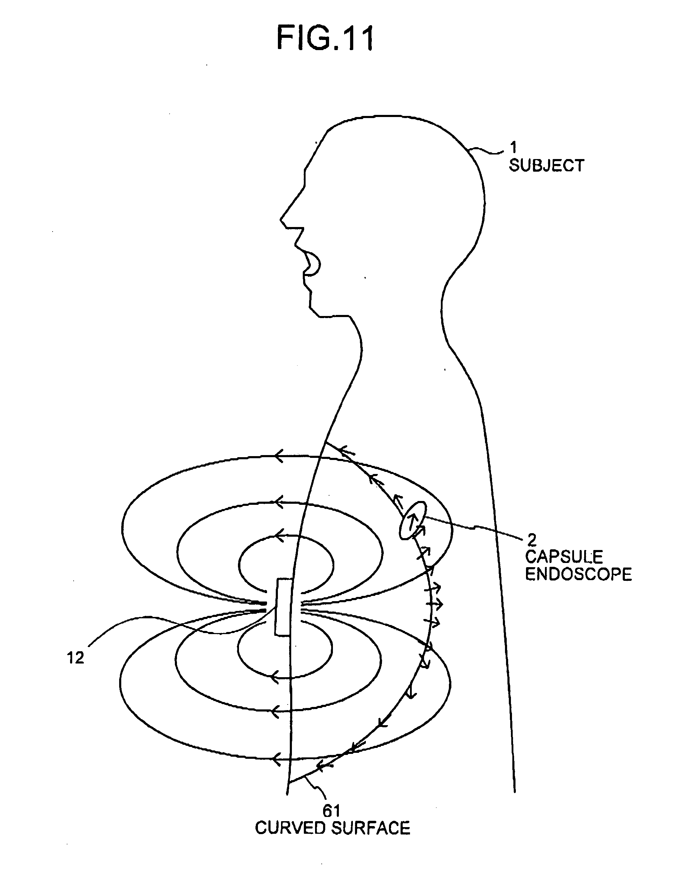

Then, the position calculator 46 calculates the position of the capsule endoscope 2 on the curved surface 61 based on the magnetic field signal S₃, the orientation information calculated by the orientation calculator 45, and the information stored in the magnetic-field line orientation database 47. Specifically, the position calculator 46 calculates the advance direction of the diffuse magnetic field at the position where the capsule endoscope 2 is present based on the magnetic field signal S₃ and the orientation information. Since the magnetic field signal S₃ is a signal corresponding to the result of detection of the diffuse magnetic field based on the target coordinate axes, when the coordinate transformation processing is performed on the advance direction of the diffuse magnetic field based on the magnetic field signal S₃ from the target coordinate axes to the reference coordinate axes with the use of the orientation information, the advance direction of the diffuse magnetic field at the position where the capsule endoscope 2 is present and on the reference coordinate axes can be calculated. Since the magnetic-field line orientation database 47 records the correspondences between the advance direction and the position of the diffuse magnetic field on the reference coordinate axes, the position calculator 46 calculates the position corresponding to the calculated advance direction of the diffuse magnetic field by referring to the information stored in the magnetic-field line orientation database 47 as shown in FIG. 11, and identifies the calculated position as the position of the capsule endoscope 2. The above is the position calculation mechanism of the position calculator 46.

Next, advantages of the body insertable system according to the first embodiment will be described. Firstly, in the body insertable system according to the first embodiment, as shown in FIGS. 1 and 3, in the receiving apparatus 3, the receiving unit 6 and the position detecting unit 7 are formed separately and independently, and therefore, the arrangement thereof relative to the subject 1 can be adjusted according to the purpose of use. For example, when the examination is carried out for the purpose of both the acquisition of the intra-subject information and the position detection, the purpose can be achieved by using the receiving apparatus 3 in a state where the receiving unit 6 and the position detecting unit 7 are combined. On the other hand, when the position detection is not necessary and the acquisition of the intra-subject information alone is the purpose of use, the intra-subject information acquired by the capsule endoscope 2 can be recorded into the portable recording medium 5 by removing the position detecting unit 7 from the subject 1 and using the receiving unit 6 alone.

Thus, the body insertable system according to the first embodiment has an advantage that the burden on the subject 1 at the use can be restricted to a minimum degree according to the purpose of use. Specifically, in the first embodiment, when the position detection is not performed, the subject 1 does not need to carry the first linear magnetic field generator 11a, the second linear magnetic field generator 11b, the diffuse magnetic field generator 12, and the processing device 13, that are used for position detection, whereby the burden on the subject 1 at the use can be alleviated.

Further, the body insertable system according to the first embodiment has an advantage that the burden on the subject 1 can be restricted to a minimum degree according to the purpose of use, while the increase in the operational cost can be suppressed. In other words, the body insertable system according to the first embodiment alone can satisfy both purposes of use, i.e., the acquisition of the intra-subject information alone, and the acquisition of the intra-subject information and the position detection, whereby the operational cost can be decreased in comparison with the cost incurred when different systems are used.

Further, with respect to the capsule endoscope 2, which is an element of the body insertable system, the reduction of the operational cost is realized. Specifically, in the first embodiment, as shown in the flowchart of FIG. 7, the magnetic field detection operation related with the position detection is not carried out unless the control signal is received from the position detecting unit 7. Therefore, when the receiving unit 6 alone is employed in the receiving apparatus 3, the magnetic field sensor 16 and the like are not driven, and thus, the power consumption is reduced by an amount of power required fro the driving of the magnetic field sensor 16 and the like, whereby the operational cost of the overall system can be reduced.

Further, the body insertable system according to the first embodiment has an advantage that the accurate position detection can be performed while the burden on the subject 1 is reduced when the body insertable system is used for position detection. As is clear from the description based on FIGS. 9 to 11, the position detection is carried out based on the advance direction and the strength of the magnetic field for position detection, and hence, the first linear magnetic field generator 11a, the second linear magnetic field generator 11b, and the diffuse magnetic field generator 12 which generate the magnetic fields for position detection are required to be fixed at given positions relative to the subject 1 until the use of the body insertable system is finished. Therefore, the first linear magnetic field generator 11a and the like are of course arranged in close contact with and fixed relative to the subject 1, and further, the first linear magnetic field generator 11a and the like are usually connected to the position detection mechanism by a cable as shown in FIG. 1, for example.

Therefore, in order to safely prevent the displacement of the first linear magnetic field generator 11a and the like at the change of posture of the subject 1, for example, the position detection mechanism which is connected to the first linear magnetic field generator 11a and the like by a cable is required to be fixed relative to the subject 1. Therefore, when the system including the receiving apparatus in which the receiving unit and the position detecting unit are integral as in the conventional system is employed, the receiving apparatus is arranged so that the receiving apparatus assumes a fixed state relative to the subject 1. However, the conventional receiving apparatus is more bulky and heavier since the receiving unit and the position detecting unit are integral, whereby the burden on the subject 1 becomes significant if such a receiving apparatus is fixed to the subject 1 and used for several hours.

On the other hand, in the first embodiment as described above, in the receiving apparatus 3, the receiving unit 6 and the position detecting unit 7 are formed separately and independently of each other, and only the position detecting unit 7 is connected to the first linear magnetic field generator 11a by a cable as shown in FIGS. 1 and 3. Therefore, in the body insertable system according to the first embodiment, elements required to be fixed relative to the subject 1 in the receiving apparatus 3 is the position detecting unit 7 alone in addition to the first linear magnetic field generator 11a and the like. Since the position detecting unit 7 is smaller and lighter than the conventional receiving apparatus in which the receiving unit 6 is integrally formed, the first embodiment allows for the accurate position detection while alleviating the burden on the subject I in comparison with the conventional system.

Specifically, it is preferable that the position detecting unit 7 be fixed to the subject 1 by a belt-like holder, for example, and the receiving unit 6 be arranged with a shoulder-strap-like holder in such a manner that the position thereof relative to the subject 1 can be changed. With such an arrangement, the degradation in the position detection accuracy can be prevented, and with respect to the receiving unit 6, the fatigue of the subject 1 can be alleviated by changing the position of the receiving unit 7 relative to the subject 1 every few hours.

### Second Embodiment

Next, a body insertable system according to a second embodiment will be described. In the second embodiment, the receiving apparatus is configured with a receiving unit and a position detecting unit that are formed separately and independently of each other, similarly to the first embodiment, and the capsule endoscope 2 is configured so as to start magnetic field detection in response to the generation of the magnetic field for position detection by the position detecting unit.

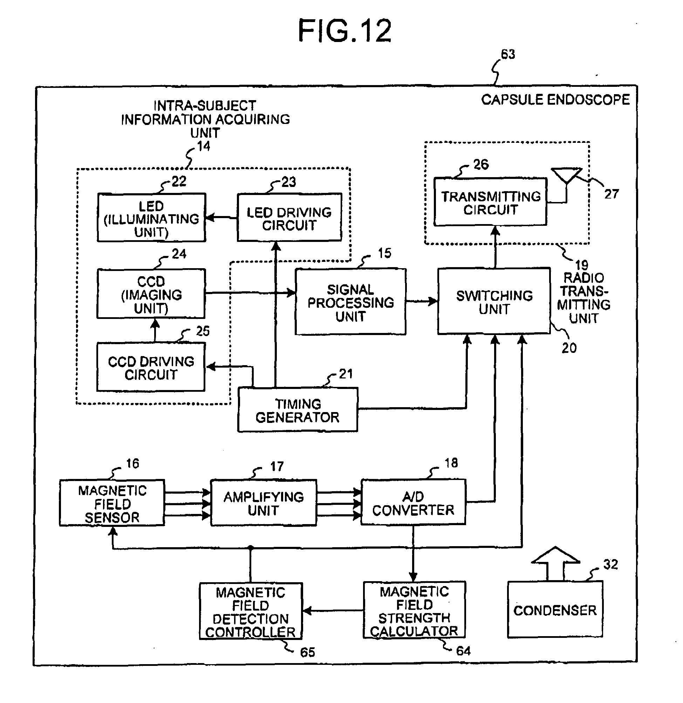

FIG. 12 is a schematic block diagram showing a structure of a capsule endoscope 63 constituting the body insertable system according to the second embodiment. Though not shown in FIG, 12 and subsequent drawings, the body insertable system according to the second embodiment includes the display device 4 and the portable recording medium 5, similarly to the first embodiment. Further, when the elements shown in FIGS. 12 and the subsequent drawings have the same reference characters and names as those in the first embodiment, they have the same structures and the same functions as those in the first embodiment, if not otherwise specified hereinbelow.

As shown in FIG. 12, the capsule endoscope 63 includes the intra-subject information acquiring unit 14, the signal processing unit 15, the magnetic field sensor 16, the amplifying unit 17, the A/D converter 18, the radio transmitting unit 19, the switching unit 20, the timing generator 21, and the condenser 32, similarly to the capsule endoscope 2 of the first embodiment, and further, additionally includes a magnetic field strength calculator 64 which calculates the strength of the detected magnetic field based on the output from the A/D converter 18, and a magnetic field detection controller 65 which controls driven states of the magnetic field sensor 16 and the switching unit 20 based on the magnetic field strength calculated by the magnetic field strength calculator 64.

The magnetic field strength calculator 64 serves to calculate the strength of the magnetic field as detected by the magnetic field sensor 16. Specifically, electric signals corresponding to the magnetic field detected by the magnetic field sensor 16 are, after being amplified by the amplifying unit 17, converted into digital signals by the A/D converter 18. The magnetic field strength calculator 64 has functions of calculating the magnetic field strength based on the digital signals obtained as a result of conversion by the A/D converter 18, and outputting the magnetic field strength to the magnetic field detection controller 65.

The magnetic field detection controller 65 has a function of controlling a period of magnetic field detection performed by the magnetic field sensor 16 based on the magnetic field strength calculated by the magnetic field strength calculator 64. Specifically, the magnetic field detection controller 65 has functions of determining whether the magnetic field for position detection is generated by the first linear magnetic field generator 11a and the like based on the magnetic field strength calculated by the magnetic field strength calculator 64, and switching the period of the magnetic field detection operation by the magnetic field sensor 16 between a long period and a short period which is shorter than the long period.

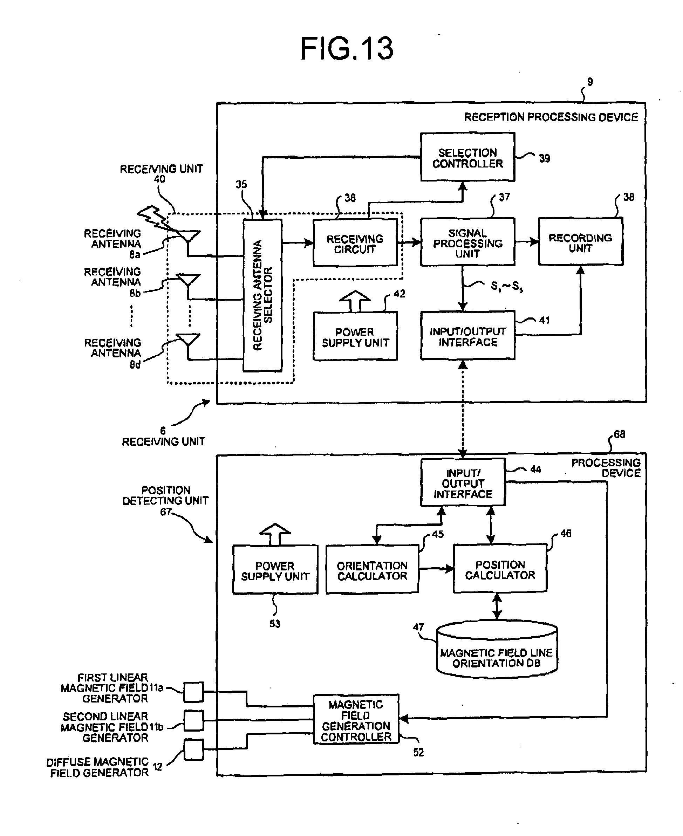

Next, a receiving apparatus constituting the body insertable system according to the second embodiment will be described. FIG. 13 is a schematic block diagram showing a structure of the receiving apparatus. As shown in FIG. 13, the receiving apparatus according to the second embodiment includes the receiving unit 6 having the same structure as the unit in the first embodiment, and a position detecting unit 67 which is formed separately and independently of the receiving unit 6 and has a different structure from the structure of the position detecting unit 7 of the first embodiment.

The position detecting unit 67 includes the first linear magnetic field generator 11a, the second linear magnetic field generator 11b, the diffuse magnetic field generator 12, and a processing device 68. The processing device 68 is configured to have, similarly to the processing device 13 of the first embodiment, the input/output interface 44, the orientation calculator 45, the position calculator 46, the magnetic-field line orientation database 47, and the power supply unit 53, and on the other hand, the control signal generator 48, the transmitting circuit 49, the transmitting antenna selector 50, and the selection controller 51 are eliminated. Corresponding to the above structure, the magnetic field generation controller 52 controls the driven states of only the first linear magnetic field generator 11a, the second linear magnetic field generator 11b, and the diffuse magnetic field generator 12, and the transmitting antennas 10a to 10d for transmitting the radio signals including the control signals in the first embodiment are eliminated.

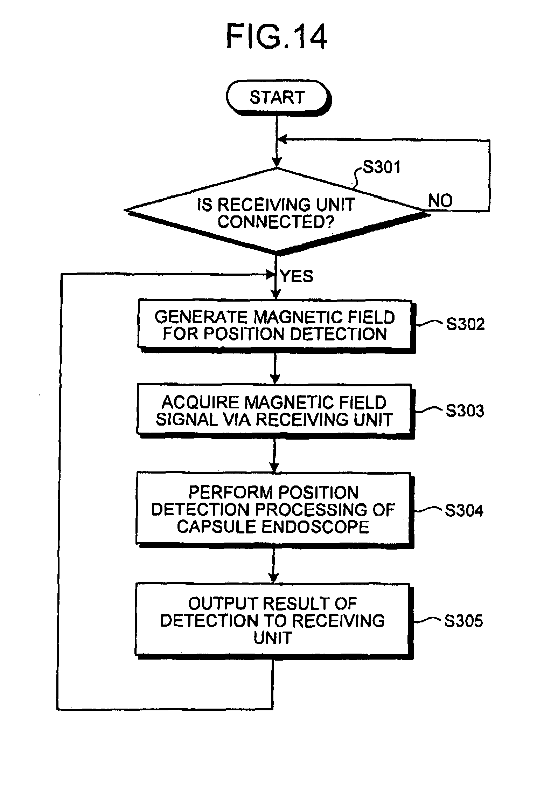

Next, an operation of the body insertable system according to the second embodiment will be described. FIG. 14 is a flowchart showing an operation of the position detecting unit 67 constituting the body insertable system. As shown in FIG. 14, the position detecting unit 67 determines whether the receiving unit 6 is connected or not by the magnetic field generation controller 52 (step S301), and, when the receiving unit 6 is connected (Yes in step S301), generates the magnetic field for position detection without performing the generation of the control signals and the like (step S302). Thereafter, the position detecting unit 67 repeats an operation, similarly to the unit of the first embodiment, of acquiring the magnetic field signals (step S303), performing the position detection processing of the capsule endoscope 2 (step S304), and outputting the result of position detection to the receiving unit 6 (step S305).

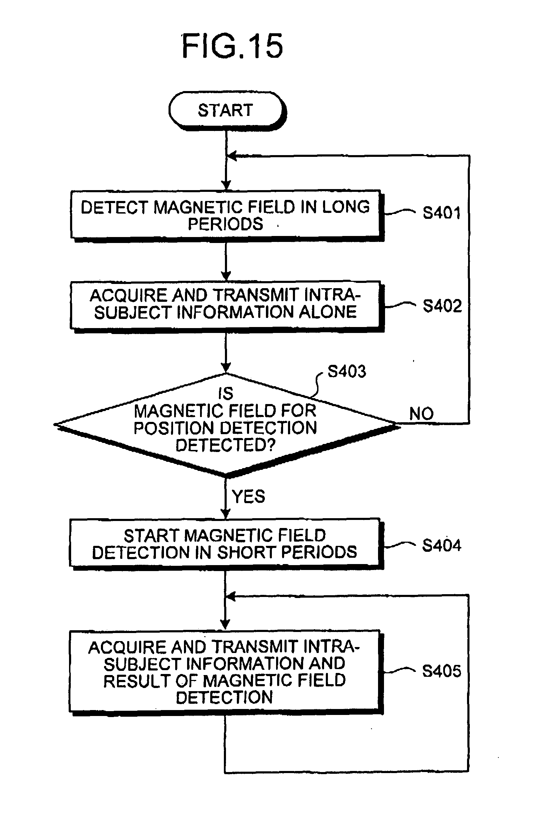

The capsule endoscope 2 operates as follows. Specifically, as shown in a flowchart of FIG. 15, the capsule endoscope 2 performs the magnetic field detection operation at long time intervals, i.e., in long periods in an initial state (step S401). Then the capsule endoscope 2 acquires the intra-subject information by the intra-subject information acquiring unit 14 and transmits radio signals including the acquired intra-subject information via the radio transmitting unit 19 (step S402). In step S402, the result of magnetic field detection in step S401 is not transmitted. Thereafter, the magnetic field detection controller 65 determines whether the magnetic field sensor 16 detects the magnetic field for position detection or not based on the detected magnetic field strength (step S403), and when the magnetic field sensor 16 does not detect the magnetic field for position detection (No in step S403), repeats the operation from step S401 assuming that the magnetic field for position detection has not been generated. On the other hand, when the magnetic field sensor 16 detects the magnetic field for position detection, the magnetic field detection controller 65 starts the magnetic field detection operation changing the detection period from the aforementioned long period to the short period, which is shorter than the long period (step S404), and repeats the transmission of radio signals including the result of magnetic field detection acquired by the magnetic field sensor 16 together with the intra-subject information acquired by the intra-subject information acquiring unit 14 (step S405).

Next, advantages of the body insertable system according to the second embodiment will be described. Firstly, in the body insertable system according to the second embodiment, similarly to the first embodiment, the receiving unit 6 and the position detecting unit 67 are formed separately and independently of each other, whereby the burden on the subject 1 can be restricted to a minimum degree according to the purpose of use, while the increase in the operational cost is prevented.

Further, the second embodiment is configured so as to detect the use of the position detecting unit 67 utilizing the magnetic field sensor 16 provided in the capsule endoscope 63. Specifically, as described above, the magnetic field sensor 16 is configured so as to perform the magnetic field detection operation by repeatedly performing the detection operation in long periods according to the control by the magnetic field detection controller 65 at a stage where it is not known whether the position detecting unit 67 is combined or not, and is configured to recognize that the position detecting unit 67 is combined according to the determination on the presence/absence of the generated magnetic field for position detection by the magnetic field detection controller 65 based on the detected magnetic field strength. Therefore, in the second embodiment, the capsule endoscope 63 does not need to include the radio receiving unit, the signal processing unit, and the like, whereby a simplified structure allows for downsizing of the capsule endoscope 63 and reduction of power consumption. Though the magnetic field sensor 16 of the second embodiment is continuously driven regardless of the generation of the magnetic field for position detection, there is no inconvenience related with a substantial increase in power consumption since the magnetic field sensor 16 is driven in long periods until the magnetic field for position detection is detected as described above.

The structure of the position detecting unit 67 is simplified as well. Specifically, since the generation and the transmission of the control signals are not necessary, the control signal generator and the transmitting unit can be eliminated from the position detecting unit 67, whereby decreases in size, weight, and power consumption are allowed. In particular, since it is desirable to arrange the position detecting unit 67 in a fixed state relative to the subject 1 for the suppression of degradation in the position detection accuracy as described with respect to the first embodiment, there is an advantage that the decrease in size and weight of the position detecting unit 67 allows for further reduction in the burden on the subject 1. Further, since the transmitting antenna constituting the transmitting unit can be eliminated, the members attached to the outer surface of the subject 1 is reduced, and the burden on the subject 1 can be alleviated in this respect as well. Third Embodiment

Next, a body insertable system according to a third embodiment will be described. The body insertable system according to the third embodiment is configured so as to perform the position detection in the position detecting unit by using earth magnetism instead of the first linear magnetic field. In the following description, a structure based on the first embodiment will be described as an example, although it is obvious that a structure using the earth magnetism in place of the first linear magnetic field can be applied to the structure of the second embodiment.

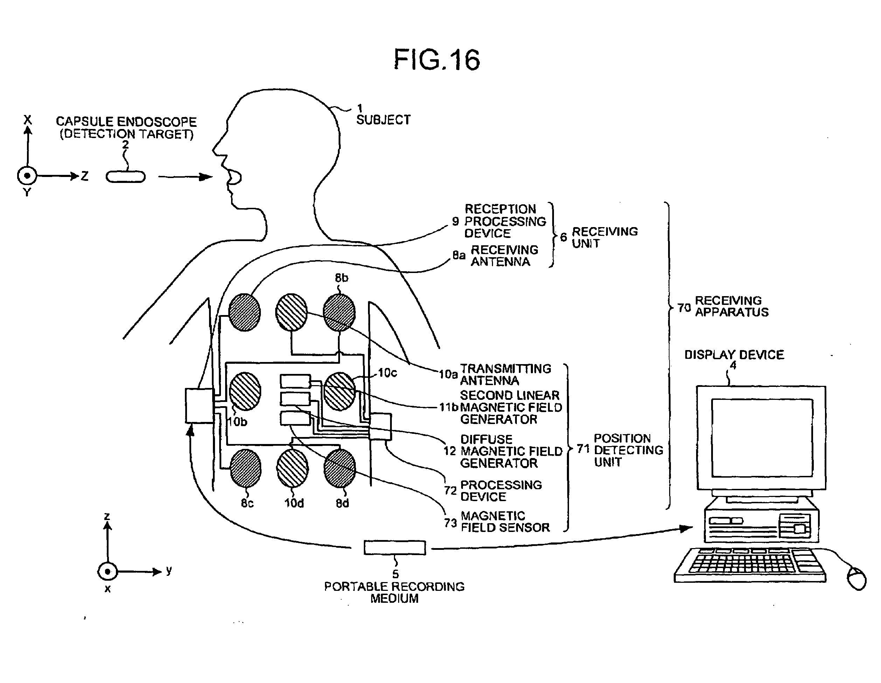

FIG. 16 is a schematic diagram showing an overall structure of the body insertable system according to the third embodiment. As shown in FIG. 16, the body insertable system according to the third embodiment includes, similarly to the first embodiment, the capsule endoscope 2, the display device 4, and the portable recording medium 5, and on the other hand, includes a position detecting unit 71 with a different structure in a receiving apparatus 70. Specifically, the first linear magnetic field generator 11a provided in the position detecting device in the first embodiment and the like is eliminated and an earth-magnetism sensor 73 is additionally provided. Further, a processing device 72 has a different structure from that of the processing device in the first embodiment and the like.

The earth-magnetism sensor 73 basically has the same structure as the magnetic field sensor 16 provided in the capsule endoscope 2. Specifically, the earth-magnetism sensor 73 has functions of detecting strength of magnetic field components in three predetermined axis directions in a region where the earth-magnetism sensor 73 is arranged, and outputting electric signals corresponding to the detected magnetic field strength. On the other hand, the earth-magnetism sensor 73 is, dissimilar to the magnetic field sensor 16, arranged on a body surface of the subject 1, and has a function of detecting the strength of the magnetic field component corresponding to each of the x-axis direction, the y-axis direction, and the z-axis direction on the reference coordinate axes fixed relative to the subject 1. In other words, the earth-magnetism sensor 73 has a function of detecting an advance direction of the earth magnetism, and is configured to output electric signals corresponding to the magnetic field strength detected in the x-axis direction, the y-axis direction, and the z-axis direction to the processing device 72.

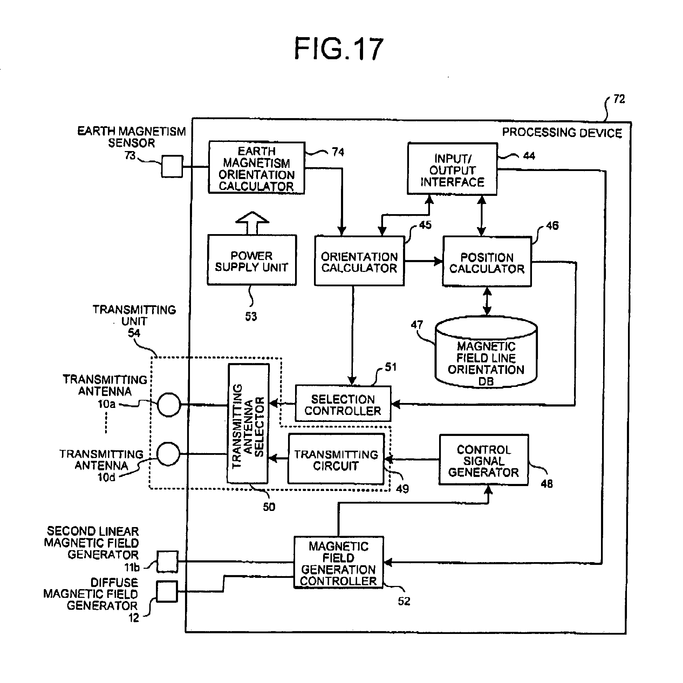

Next, the processing device 72 according to the third embodiment will be described. FIG. 17 is a block diagram of a structure of the processing device 72. As shown in FIG. 17, the processing device 72 basically has the same structure as that of the processing device 13 according to the first embodiment, and on the other hand, the processing device 72 includes an earth-magnetism orientation calculator 74 which calculates the advance direction of the earth magnetism on the reference coordinate axes based on the electric signals input from the earth-magnetism sensor 73 and outputs the result of calculation to the orientation calculator 45.

When the earth magnetism is utilized as the first linear magnetic field, the calculation of the advance direction of the earth magnetism on the reference coordinate axes fixed relative to the subject 1 is problematic. Since the subject 1 can freely move while the capsule endoscope 2 travels through inside the body, positional relations between the reference coordinate axes fixed relative to the subject 1 and the earth magnetism are expected to fluctuate along with the movement of the subject 1. On the other hand, for the calculation of the positional relations between the target coordinate axes relative to the reference coordinate axes, it is problematic that the correspondence between the reference coordinate axes and the target coordinate axes cannot be made clear with respect to the advance direction of the first linear magnetic field, when the advance direction of the first linear magnetic field on the reference coordinate axes become unknown.

Therefore, in the third embodiment, the earth-magnetism sensor 73 and the earth-magnetism orientation calculator 74 are provided to monitor the advance direction of the earth magnetism which varies on the reference coordinate axes due to the movements of the subject 1, for example. Specifically, the earth-magnetism orientation calculator 74 calculates the advance direction of the earth magnetism on the reference coordinate axes based on the result of detection by the earth-magnetism sensor 73, and outputs the result of calculation to the orientation calculator 45. In response, the orientation calculator 45 calculates the correspondence between the reference coordinate axes and the target coordinate axes with respect to the advance direction of the earth magnetism using the input advance direction of the earth magnetism, thereby allowing the calculation of the orientation information together'with correspondence with respect to the second linear magnetic field.

Depending on the direction of the subject 1, the advance direction of the earth magnetism may be parallel to the second linear magnetic field generated by the second linear magnetic field generator 11b. The detection of the position relation is still possible with the use of data concerning the orientation of the'target coordinate axes and a position of an origin of the target coordinate axes at an immediately previous time point. Further, it is effective to make the coil 34 constituting the second linear magnetic field generator 11b extend not in the y-axis direction on the reference coordinate axes as shown in FIG. 3 but in the z-axis direction, in order to prevent the earth magnetism from becoming parallel to the second linear magnetic field.

Next, advantages of the body insertable system according to the third embodiment will be described. The body insertable system according to the third embodiment has further advantages attributable to the use of the earth magnetism, in addition to the advantages of the first embodiment. When the structure utilizing the earth magnetism as the first linear magnetic field is adopted, a mechanism for generating the first linear magnetic field can be eliminated, whereby it is possible to calculate the positional relations of the target coordinate axes relative to the reference coordinate axes while alleviating the burden on the subject 1 at the introduction of the capsule endoscope 2. Since the earth-magnetism sensor 73 can be configured with an MI sensor or the like, the downsizing is well possible, and the addition of the earth-magnetism sensor 73 would not cause the increase in the burden on the subject 1.

Further, the structure utilizing the earth magnetism as the first linear magnetic field is advantageous in terms of reduction of power consumption. When the first linear magnetic field is generated by the coil or the like, the amount of consumed power increases due to the electric current flow through the coil. The use of the earth magnetism eliminates the need of such power consumption, whereby a system with low power consumption can be realized.

In the above, the present invention is described with reference to the first to the third embodiments. The present invention, however, should not be interpreted as to be limited to the above embodiments, and those skilled in the art can reach various embodiments and modifications. For example, the capsule endoscope as the body insertable apparatus in the first to the third embodiments is described as the structure having a function of acquiring the intra-subject information and a function of detecting the magnetic field for position detection as necessary in a single structure, however, as a more simple structure, a body insertable apparatus which can only acquire intra-subject information and a body insertable apparatus which is provided with both the function of acquiring the intra-subject information and the function of detecting the magnetic field for position detection may be separately prepared. Further, though the receiving apparatus is described as being provided with the power supply unit or the electric current source corresponding to each element in the above, the power supply unit provided in the receiving unit, for example, may be configured so as to supply driving power to each element, or alternatively, a battery park or the like formed separately and independently of the receiving unit and the like may be employed to supply driving power to the receiving unit and the like.

### INDUSTRIAL APPLICABILITY

As can be seen from the foregoing, the body insertable system, the receiving apparatus, and the body insertable apparatus according to the present invention are useful for a medical observation apparatus which is introduced inside a human body and employed for an observation of an examined area, and in particular, is suitable for restricting a burden of a subject at the use to a minimum degree according to the purpose of use while suppressing the increase in the operational cost, with respect to the body insertable system provided with the body insertable apparatus such as the capsule endoscope.

## Claims

1. A body insertable system including a body insertable apparatus that is introduced inside a subject, acquires intra-subject information as information concerning the subject, and transmits radio signals including the acquired intra-subject information, and a receiving apparatus that performs reception processing of the radio signals transmitted by the body insertable apparatus,
the body insertable apparatus comprising:
an intra-subject information acquiring unit that acquires the intra-subject information;
a magnetic field sensor that detects a magnetic field in a region where the body insertable apparatus is located; and
a radio transmitting unit that transmits radio signals including at least the intra-subject information, and
the receiving apparatus comprising:
a receiving unit that includes at least a receiving antenna which receives the radio signals transmitted by the body insertable apparatus and a receiving circuit which performs reception processing on the radio signals received by the receiving antenna; and
a position detecting unit that includes a magnetic field generator which generates a predetermined magnetic field for position detection in a region where the body insertable apparatus can be present, and a position calculator that calculates a position of the body insertable apparatus based on a result of detection of the magnetic field for position detection acquired by the magnetic field sensor, the position detecting unit being formed separately and independently of the receiving unit.

2. The body insertable system according to claim 1, wherein
the radio transmitting unit transmits radio signals including a result of detection acquired by the magnetic field sensor in addition to the intra-subject information, and
the position detecting unit acquires the result of detection acquired by the magnetic field sensor via the receiving unit.

3. The body insertable system according to claim 1 or 2, wherein
the position detecting unit is arranged in a fixed state relative to the subject at a time of use, and
the receiving unit is arranged in a movable state relative to the subject at a time of use.

4. A receiving apparatus performing reception processing of a radio signal transmitted from a predetermined detection target, comprising:
a receiving unit that includes at least a receiving antenna which receives the radio signal transmitted from the detection target, and a receiving circuit which performs reception processing on the radio signal received by the receiving antenna; and
a position detecting unit that includes a magnetic field generator which generates a predetermined magnetic field for position detection in a region where the detection target can be present, and a position calculator which calculates a position of the detection target based on a result of detection of the magnetic field for position detection in the region where the detection target can be present, the position detecting unit being formed separately and independently of the receiving unit.

5. A body insertable apparatus introduced inside a subject and acquiring intra-subject information as information concerning the subject, comprising:
an intra-subject information acquiring unit that acquires the intra-subject information;
a magnetic field sensor that detects a magnetic field in a region where the body insertable apparatus is located;
a radio transmitting unit that transmits radio signals including at least the intra-subject information; and
a magnetic field detection controller that controls a driven state of the magnetic field sensor.

6. The body insertable apparatus according to claim 5, further comprising:
a radio receiving unit that receives a radio signal transmitted from an outside, wherein
the magnetic field detection controller controls the driven state of the magnetic field sensor based on a control signal received by the radio receiving unit.

7. The body insertable apparatus according to claim 5, wherein
the magnetic field sensor performs a magnetic field detection in a stand-by mode in which a detection interval is longer than in a normal mode when the magnetic field for position detection is not generated in the region where the body insertable apparatus is located, and transits from the stand-by mode to the normal mode when the magnetic field for position detection is detected during the stand-by mode.
